(19) Europäisches Patentamt
European Patent Office
Office européen des brevets

(11) Publication number: 0 429 464 B1

(12) # EUROPEAN PATENT SPECIFICATION

(45) Date of publication of patent specification: 23.11.94

(51) Int. Cl.5: C07D 493/04, A61K 31/35, C07D 498/04, A61K 31/395, A61K 31/34, //(C07D311/00, 307:00)

(21) Application number: 89907579.0

(22) Date of filing: 16.06.89

(86) International application number: PCT/US89/02572

(87) International publication number: WO 90/02129 (08.03.90 90/06)

(54) BIVALENT LIGANDS EFFECTIVE FOR BLOCKING ACAT ENZYME.

(30) Priority: 16.08.88 US 232931

(43) Date of publication of application: 05.06.91 Bulletin 91/23

(45) Publication of the grant of the patent: 23.11.94 Bulletin 94/47

(84) Designated Contracting States: AT BE CH DE FR GB IT LI LU NL SE

(56) References cited:
EP-A- 0 095 836
GB-A- 2 043 059

Chemical Abstracts, vol. 100, 1984 (Columbus Ohio, US), F. Eiden et al.: "Pyran derivatives. Part 101. Reactions of 6-acyl-7-norkhellin derivatives with amines", see page 601, abstract no. 174494n, & Arch. Pharm. (Weinheim, Ger.) 1984, 317 (3), 203-7

(73) Proprietor: THE UPJOHN COMPANY
301 Henrietta Street
Kalamazoo, Michigan 49001 (US)

(72) Inventor: GAMMILL, Ronald, B.
6704 Pleasantview Drive
Portage, MI 49081 (US)
Inventor: BELL, Frank, P.
12531 Sprinkle Road
Vicksburg, MI 49097 (US)

(74) Representative: Perry, Robert Edward et al
GILL JENNINGS & EVERY
Broadgate House
7 Eldon Street
London EC2M 7LH (GB)

EP 0 429 464 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed toward new bivalent ligands effective for blocking or inhibiting acyl-CoA: cholesterol O-acyltransferase enzyme (hereinafter, ACAT) which is a major regulator of cholesterol metabolism in cells. The blocking or inhibition of ACAT is useful in the prevention or treatment of a variety of physiological conditions associated with arterial vessels. The compounds of the present invention are paticularly useful in the prevention or treatment of the constriction or obstruction of arteries and atherosclerosis.

ACAT is found, in most tissues including arterial, liver, adrenal gland, mammary gland, ovaries and intestine where it readily converts cholesterol into esterified cholesterol. Bell, F.P., Arterial Cholesterol Esterification By AcylCoAcholesterol Acyltransferase: Significance in Atherogenesis and its Inhibition by Drugs, Pharmacological Control of Hyperlipidaemia, JR Prous Sci. Pub., pp 409-22 (1986). Generally this reaction is in equilibrium with a hydrolysis reaction which converts the esterified cholesterol into cholesterol. The amount of available cholesterol which effects the balance of this equilibrium is dependent on many physiological factors and diet. Unfortunately, esterified cholesterol does not migrate through tissue as easily as cholesterol and can build-up and form obstructions. The accumulation of esterified cholesterol is one of the characteristic features of atherosclerotic plaque. Therefore it would be of great advantage if the ACAT enzyme could be blocked or inhibited from turning cholesterol into esterified cholesterol in arterial tissues.

### Information Disclosure

The literature discuss the use of ACAT inhibitors as potential antiatherosclerotic agents as disclosed in V.G. DeVries, et al., J. Med. Chem., 29, 1131 (1986) and J. Med. Chem., 26, 1411 (1983). Also, the role of acyl-CoA:cholesterolacyltransferase in cellular cholesterol metabolism is discussed in K.E. Suckling and E.F. Stange, J. Lipid Res., 26, 647 (1985).

The subject bivalent ligands are represented by $\alpha$-$\beta$-$\alpha$ where $\beta$ is a chemical tether connecting two heterocyclic groups $\alpha$. The heterocyclic groups are furochromones, furobenzoxazines and benzodifurans which are generally disclosed in U.S. Patents 4,284,569, 4,412,071

The present invention is directed to a family of bivalent ligand compounds $\alpha$-$\beta$-$\alpha$ formed from two heterocyclic compounds "$\alpha$" connected by a tether "$\beta$", structurally represented on the formula sheet below, wherein the variables are as defined in claim 1.

The preferred tethers are derived from trimethylene-4,4-dipiperidine or 1,2-ethanediyl-4,4-dipiperidine. One example of a bivalent ligand is 7,7'-[1,2-ethanediylbis(4,1-piperidinediylmethylene)]-bis[4,9-dimethyl-5H-furo[3,2-g][1]benzopyran-5-one].

The bivalent ligands of the invention are useful for blocking or inhibiting ACAT enzyme, or preventing or treating atherosclerosis, by administering a pharmacological amount of the compound or an acceptable salt thereof to a subject including humans. The blocking or inhibition of ACAT is useful in the prevention or treatment of a variety of physiological conditions associated with arterial vessels. This is particularly suitable for administration subsequent to by-pass surgery, coronary by-pass surgery, angioplasty or transplants.

### Detailed Description of the Invention

The compounds of the present invention are bivalent ligands, represented by "$\alpha$-$\beta$-$\alpha$", such as bisaminofurochromones and bisaminobenzodifurans formed from two heterocyclic ring structures "$\alpha$" linked by a chemical tether "$\beta$". The heterocyclic compounds $\alpha$-Z, wherein Z is a primary, secondary or tertiary amine, i.e. furochromones, furobenzoxazines and benzodifurans, and their syntheses are disclosed in U.S. Patents 4,284,569, 4,412,071 and 4,304,722, where they are reported to be antiatherogenic compounds having antiatherosclerosis activity.

Examples of counter-ions are $Li^+$, $Na^+$, $K^+$ and $Ca^{2+}$.

Examples of "alkyl" are methyl, ethyl, propyl, butyl, pentyl, hexyl, heptyl, octyl and isomeric forms thereof.

Examples of "aryl" are phenyl, $\alpha$-naphthyl, $\beta$-naphthyl, m-methylphenyl and p-trifluoromethylphenyl.

Examples of "cycloalkyl" are cyclopropyl, cyclobutyl, cyclopentyl, cyclohexyl, cycloheptyl and cyclodecyl.

Examples of "alkylaryl" are alkyl chains of one to 8 carbon atoms and isomeric forms thereof which are substituted with aryl groups of 6 to 12 carbon atoms as described above.

Examples of "heteroaryl" are pyridine, thiophene, furan and pyrmidine.

Examples of "heteroalkyl" are one to 8 carbon atoms alkyls as described above which contain heteroatoms such as nitrogen, sulfur and oxygen.

Examples of "halo" are fluorine, chlorine, bromine and iodide.

Connecting two of the heterocyclic groups "$\alpha$" with one of the tethers "$\beta$" produces an inhibitor of ACAT. Generally the heterocyclic group is the same at both ends of the tether however the bivalent ligands can comprise a different heterocyclic group at either end of the tether. Regardless, tethering two heterocyclic structures together provides greater potency, and the heteroatoms in the tether appear to improve the interaction of the bivalent ligand with the tissue for inhibiting ACAT.

Various chemical schemes for preparing these compounds are described below. The specific structures for compounds prepared according to this invention are shown in Tables 1 and 2 along with their measured inhibition of ACAT at various levels.

The ratio of $^3$H-cholesteryl ester radioactivity to the sum of the $^3$H-cholesterol plus $^3$H-cholesteryl ester recovered in the cellular extracts X 100 yields the percent of total $^3$H-cholesterol taken up which was esterified by cellular ACAT and is referred to as percent ACAT. Percent inhibition of ACAT is also mathematically derived from the data for convenience. Values for % ACAT that are less than control values identify assay cultures in which ACAT was inhibited; the positive standard values provide a basis for relative potency evaluation. Control values for percent ACAT typically range form 60-70% under the test conditions employed.

Compound I (Table 1) is prepared by the treatment of C-7 methylthiomethylsulfide with excess methyl iodide in methylene chloride at reflux for 72 hours to produce the allylic iodide form in a 70 to 75% yield as a pale yellow solid. This product is then treated with trimethylene-4,4-piperidine in acetonitrile in the presence of potassium carbonate to yield the allylic bisamine form, i.e., Compound I, as a powdery solid.

The synthesis of the bisaminofurobenzoxazinone, Compound II (Table I) is performed by oxidation of khellin with basic hydrogen peroxide followed by esterification of the resulting acid which affords the hydroxyester. See, U.S. Patent No. 4,412,071. Treatment of the hydroxy ester with cyanogen bromide in the presence of triethylamine (TEA) in acetonitrile yields the cyanoether. The cyanoether is then treated with trimethylene-4,4-piperidine in acetonitrile, to yield the bisaminofurobenzoxazinone, i.e., Compound II.

The synthesis of the benzodifurans, Compounds III (Table 1) (see, Example 1) is accomplished by addition of diamines to bromofurochromone or bromochromone, respectively. The addition is performed in the presence of potassium carbonate and acetonitrile.

Compounds I-III are prepared with the trimethylene-4,4-dipiperidine tether and their structure and percent ACAT inhibition in micrograms per milliliter ($\mu$g/ml) are shown in Table 1. Included in Table 1 is the single heterocyclic compound IV corresponding to Compound I which shows reduced percent ACAT inhibition and demonstrates the enhanced potency observed in the corresponding bivalent ligand Compound I.

Table 2 shows the effect of the dimethylene-4,4-dipiperidine tether.

The synthesis of the dihydrofurochromone-containing Compound V (Table 2) begins with khellinone, a basic hydrolysis product of khellin. Hydrogenation of khellinone proceeds quantitatively to give a product which is then subjected to Claisen condensation with ethyl ($\alpha$-thiomethyl) acetate followed by acid-catalyzed cyclodehydration to yield dihydrofurochromone. Treatment of the dihydrofurochromone with methyl iodide and methylene chloride at reflux affords the desired allylic iodide. Treatment of the allylic iodide with dimethylene-4,4-piperidine yields the dihydro analog, Compound V.

The trimethylsilyl analog, Compound VI (Table 2) (see Example 4) is prepared in the following manner. Treatment of timefurone with two equivalents of lithium diisopropylamide (LDA) results in the formation of a dilithio species which when treated with trimethylsilyl chloride and subjected to an aqueous workup affords the 2-trimethylsilyl analog in good yield. Treatment of this product with excess methyl iodide in methylene chloride affords the allylic iodide. Addition of dimethylene-4,4-piperidine to the allylic iodide yields the desired bisaminofurochromone, Compound VI in 75.5% yield.

The present invention has identified classes of compounds which are structurally unique inhibitors of ACAT. The data indicates the preferred compounds are those such as Compound Ia (Table 2), a bisaminofurochromone.

These compounds have been shown to also exhibit antiatherosclerotic activity in the SEA Japanese quail model and Netherland Dwarf rabbit. For example, five to six week old, male, SEA quails were placed on a high cholesterol diet with one group orally receiving the subject ACAT inhibitor compound Ia (Table 2). After eight weeks the arteries were removed, cleaned and homogenized. Total cholesterol, free cholesterol and triglycerides were measured. The results were statistically analyzed and showed a significant (thirty percent) reduction in the accumulation of esterified cholesterol in the arteries for quails that received the ACAT inhibitor compound.

In another experiment, ACAT inhibitor compound Ia was administered for twelve weeks at 50 mg/kg/day to Netherland dwarf rabbits that were feed a cholesterol-containing atherogenic diet. During the twelve week period serum cholesterol, triglycerides, and carnitine were monitored. At the end of the study the aortas from the drug treated group and a non-drug treated group were examined for atherosclerosis development.

There was less extensive development of hypercholesterolemia in the treated group. The mean serum cholesterol levels in the control exceeded 2200 mg/dl whereas the mean levels in the treated group was 271 mg/dl. The lower serum cholesterol level was associated with negligible atheromatous lesion development in the treated groups. In contrast, atheromatous lesion development in the controls was extensive.

It has been concluded that the compounds of the present invention are pharmacologically effective in the reduction of esterified cholesterol not only in the general prevention or treatment of cholesterol levels but also in other physiological conditions associated with the occlusion or obstruction of arteries. For example, the subject bivalent ligands can be useful in preventing arterial occlusion in vascular trauma associated with procedures such as by-pass grafts, coronary by-passes, angioplasty and transplants.

The dosage of the bivalent ligand compound used in treatment depends on the particular use, frequency of administration and the age or condition of the recipient. Thus, the subject compounds along with any carriers or buffers would be administrated in a pharmacological amount effective to inhibit ACAT enzyme as prescribed with respect to the physiological condition diagnosed such as atherosclerosis, high blood cholesterol, artery occulsion or restriction, or surgical procedure as well as factors such as diet. Generally, the compounds can be administered in an amount of from about 0.1 to about 1000.0 mg/kg per dose.

The compounds can be administered intravenously, intramuscularly, topically, transdermally such as by skin patches, bucally or orally to man or other animals. The compositions of the present invention can be presented for administration to humans and animals in unit dosage forms, such as tablets, capsules, pills, powders, granules, sterile parenteral solutions or suspensions, oral solutions or suspensions, oil in water and water in oil emulsions containing suitable quantities of the compound, suppositories and in fluid suspensions or solutions.

For oral administration, either solid or fluid unit dosage forms can be prepared. For preparing solid compositions such as tablets, the compound can be mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by mixing the compound with an inert pharmaceutical diluent and filling the mixture into a hard gelatin capsule of appropriate size. Soft gelatin capsules are prepared by machine encapsulation of a slurry of the compound with an acceptable vegetable oil, light liquid petrolatum or other insert oil.

Fluid unit dosage forms for oral administration such as syrups, elixirs, and suspensions can be prepared. The forms can be dissolved in an aqueous vehicle together with sugar, aromatic flavoring agents and preservatives to form a syrup. Suspensions can be prepared with an aqueous vehicle with the aid of a suspending agent such as acacia, tragacanth, methylcellulose and the like.

For parenteral administration, fluid unit dosage forms can be prepared utilizing the compound and a sterile vehicle. In preparing solutions the compound can be dissolved in water for injection and filter sterilized before filling into a suitable vial or ampoule and sealing. Adjuvants such as a local anesthetic, preservative and buffering agents can be dissolved in the vehicle. The composition can be frozen after filling into a vial and the water removed under vacuum. The dry lyophilized powder can then be sealed in the vial and reconstituted prior to use.

The following examples further demonstrate in greater detail the preparation of the bivalent ligands of the subject invention.

Example 1 2,2'-[1,3-Propanediylbis(4,1-piperidinediylmethylidyne)]bis[4,8-dimethoxybenzo[1,2-b;5,4-b']-difuran-3(2H)-one (Compound III, Table 1)

6-Bromofurochromone (6.50 g, 20.0 mmol), trimethylene-4,4-piperidine (2.1 g, 10 mmol) and potassium carbonate (5.52 g, 40.0 mmol) are added to acetonitrile (100 ml) and heated at 60°C for 6 hours. The reaction is cooled to room temperature and diluted with water and vigorously stirred for 5-10 minutes. The solid that filled the flask is collected on a filter and air dried to yield 5.68 g (81.4%) the product as a brick red solid. An analytical sample is prepared by three recrystallizations form $CHCl_3/CH_3CN$ and drying the compound in a heating pistol. These recrystallizations remove the red color and small amounts of a more polar impurity to yield the biligand compound in relatively pure form.

Physical characteristics are as follows:

MP: 246°C.

| | | | |
|---|---|---|---|
| Anal. Calc'd for $C_{39}H_{42}N_2O_{10}$: | C, 67.04; | H, 6.01; | N, 4.01. |
| Found: | C, 66.70; | H, 6.13; | N, 4.08. |

Example 2 7,7'-[1,2-Ethanediylbis(4,1-piperidinediylmethylene)]bis[4,9-dimethoxy-5H-furo[3,2-g][1]-benzopyran-5-one (Compound Ia, Table 2)

The allylic iodide (50 g, 129 mmol) is added to a mixture of $CH_2Cl_2$ (125 ml) and $CH_3OH$ (200 ml). To that solution is then added triethylamine (13.03 g, 129 mmol) followed by the bisamine (10.6 g, [92% pure], 49.7 mmol) in methanol (125 ml) dropwise and the resulting reaction stirred at room temperature overnight. The reaction is then diluted with methanol (2.5 l) and the resulting solid collected on a filter to give 26.2 g, 76% of analytically pure biligand compound.

Physical characteristics are as follows:

MP: 177-79 °C (can be recrystallized from $CH_3CN$).

| | | | |
|---|---|---|---|
| Anal. Calc'd for $C_{40}H_{44}N_2O2_{10}$: | C, 67.40; | H, 6.22; | N, 3.93. |
| Found: | C, 67.21; | H, 6.31; | N, 3.84. |

Example 3 7,7'-[1,2-Ethanediylbis(4,1-piperidinediylmethylene)]bis[2,3-dihydro-4,9-dimethoxy-5H-furo[3.2-g]-[1]benzopyran-5-one] (Compound V, Table 2)

NaH ({50% oil dispersion}, 13.1 g, 272 mmol) is added to a 1 l three-neck round-bottom flask. The material is thoroughly washed with hexane and the hexane replaced with THF (300 ml). 2,3-Dihydrokhellinone (19.0 g, 79.8 mmol) is dissolved in ethyl thiomethylacetate (180 ml) and the solution is added dropwise over one hour to the NaH/THF slurry. There is a slight exotherm. The reaction is stirred at room temperature for two hours at which time TLC (5% EtOAc/$CH_2Cl_2$) indicates that the condensation reaction is complete. The solvent is removed in vacuo and the resulting oil is diluted with an equal volume of $CH_2Cl_2$ and poured into $CH_2Cl_2$, saturated with anhydrous HCl and stirred at room temperature for five hours. The reaction is evaporated in vacuo, washed with water and chromatographed over 800 g of silica gel. The column affords 8.2 g (33.4%) of the 4,9-dimethoxy-2,3-dihydro-[(7-methylthio)methyl]-5H-furo[3,2-b]-benzopyran-5-one as a tan solid.

The tan product (6.0 g, 19.5 mmol) is dissolved in a mixture of $CH_2Cl_2/CH_3I$ (1/3; 20 ml) and heated at reflux for 65 hours. The reaction is evaporated in vacuo and triturated with $CH_2Cl_2$ (4x), discarding the solid each time (product is in the organic filtrate). The solvent is finally removed in vacuo to yield 6.25 g of 4,9-dimethoxy-2,3-dihydro-[(7-methylthio)methyl]-5H-furo[3,2-b]-benzopyran-5-one, which is used without further purification.

To a $CH_2Cl_2/CH_3OH$ mixture of the allylic iodide (2.5 g, 6.44 mmol) is added triethylamine (0.54 g, 5.38 mmol). The bisamine (0.53 g, 2.69 mmol [92% pure]) is then added in $CH_3OH$ dropwise over several minutes. After stirring at room temperature overnight, the reaction is filtered and the solid washed with $CH_3OH$ and dried to yield 0.93 g of pure product. Analytical sample is prepared from $CH_3OH/CH_2Cl_2$.

Physical characteristics are as follows:

MP: 204-6 °C.

| | | | |
|---|---|---|---|
| Anal. Calc'd for $C_{40}N_{48}N_2O_{10}$: | C, 67.02; | H, 6.75; | N, 3.91. |
| Found: | C, 66.73; | H, 6.73; | N, 3.88. |

Example 4 7,7'-[1,2-Ethanediylbis(4,1-piperidinediylmethylene)]bis[4,9-dimethoxy-2-(trimethylsilyl)-5H-furo-[3,2g][1]benzopyran-5-one] (Compound VI, Table 2)

4,9-dimethoxy-7-methyl-2-(trimethylsilyl)-5H-furo(3,2-g)(1)benzopyran-5-one (5.0 g, 13.2 mmol) is dissolved in methylene chloride (20 ml) and then diluted with methyl iodide (75 g, 530 mmol). The mixture is refluxed for four days. After cooling to room temperature, the reaction is filtered and excess methyl iodide and methylene chloride removed in vacuo. This affords 4.7 g of 4,9-dimethoxy-2-trimethylsilyl-7-iodomethyl-5H-furo[3,2-b]-benzopyran-5-one of sufficient purity for use in the next step.

The allylic iodide is dissolved in a mixture of $CH_2Cl_2$ and $CH_3OH$ (10/15 ml). To that solution is added the bisamine in $CH_3OH$ (5 ml) and the reaction is stirred at room temperature overnight. The reaction is evaporated in vacuo and the resulting solid is slurried with cold $CH_3OH$ and filtered to afford 1.70 g (75.5%) of the biligand compound. An analytical sample is prepared by recrystallization from $CH_3OH/CH_2Cl_2$.

Physical characteristics are as follows:

MP: 84-7 °C.

| | | | |
|---|---|---|---|
| Anal. Calc'd for $C_{46}H_{60}N_2O_{10}Si_2$: | C, 64.46; | H, 7.06; | N, 3.27. |
| Found: | C, 64.07; | H, 6.98; | N, 3.31. |

TABLE 1

Trimethylene-4,4-dipiperidine Tether

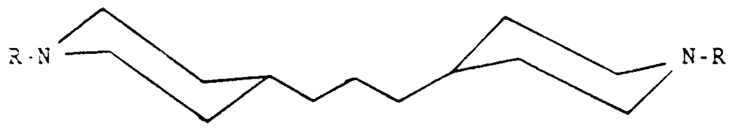

| Compound | R | % ACAT Inhibition ($\mu$g/ml) | | $IC_{50}$ |
|---|---|---|---|---|
| I | 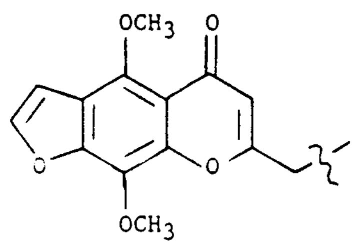 | 5 | 68% | 4.2 $\mu$g/ml |
| | | 10 | 84% | |
| | | 15 | 91% | |
| II | 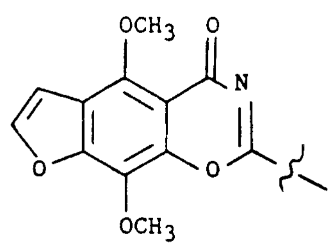 | 5 | 60% | 1.8 $\mu$g/ml |
| | | 10 | 89% | |
| | | 15 | 97% | |
| III | 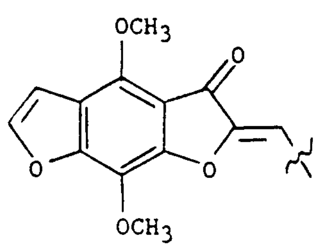 | 5 | 18% | |
| | | 10 | 29% | |
| | | 15 | 52% | |
| * IV | 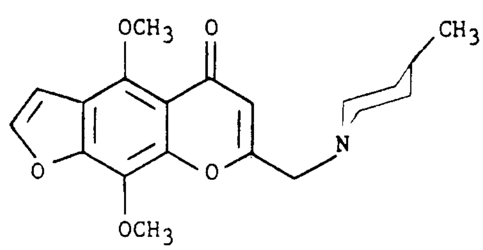 | 5 | 10% | |
| | | 10 | 27% | |
| | | 15 | 39% | |

*Not a bivalent ligand of the subject invention

TABLE 2

1,2-Ethanediyl-(4,4-dipiperidine) Tether

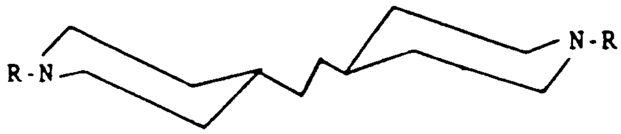

| Compound | R | % ACAT Inhibition ($\mu$g/ml) | | $IC_{50}$ |
|---|---|---|---|---|
| V | 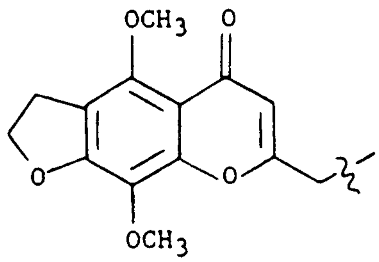 | 5 | 63% | 3.0 $\mu$g/ml |
| | | 10 | 73% | |
| | | 15 | 74% | |
| VI | 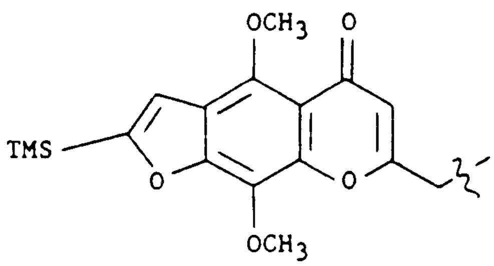 | 5 | 18% | |
| | | 10 | 29% | |
| | | 15 | 52% | |
| Ia | as I | 5 | 87% | 0.8 $\mu$g/ml |
| | | 10 | 84% | |
| | | 15 | 75% | |

HETEROCYCLIC COMPOUNDS "α"

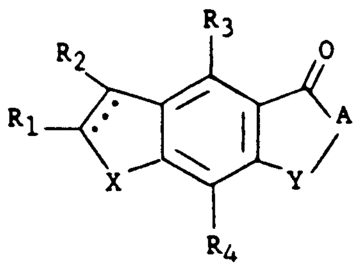

TETHER "B"

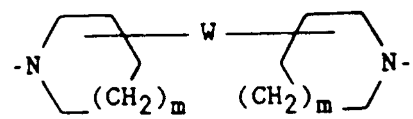

## Claims

1. A compound comprising: a bivalent ligand α-β-α wherein α is structurally represented by the formula

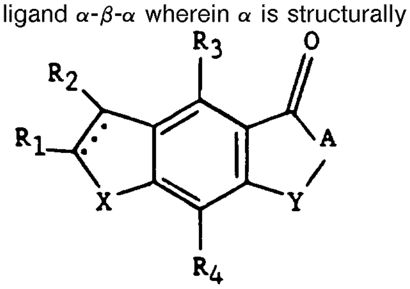

wherein:

X and Y are independently O or S;

A is C=CH- , N=C- or $CR_5=C-(CH_2)_n-$ and is linked to β;

$R_1$ and $R_2$ are independently:

a) H,

b) halo,

c) alkyl,

d) $-(CH_2)_p$-aryl,

e) $-(CH_2)_p$-heteroaryl,

f) $-(CH_2)_p-CO_2R_6$,

g) $-(CH_2)_p-CONR_7R_8$,

h) $-Si(R_9)$,

i) $-(CH_2)_n-NR_7R_8$,

j) $-(CH_2)_n-OR_{10}$,

k) $-CF_3$, or

l) $-(CH_2)_n-SR_6$, $-(CH_2)_n-SOR_6$, $-(CH_2)_n-SO_2R_6$;

$R_3$ is

a) OH,

b) $OCH_2CH=CH_2$,
c) $OCH_2CH(OH)CH_2NHR_6$,
d) -O-alkyl,
e) $-O-(CH_2)_n-CO_2R_6$, or
f) $-O-(CH_2)_n-CONR_7R_8$;

$R_4$ is
a) hydrogen,
b) halo,
c) $NO_2$,
d) $NH_2$,
e) $CF_3$,
f) alkyl,
g) aryl,
h) -S-alkyl or aryl,
i) -SO-alkyl or aryl,
j) $-SO_2$-alkyl or aryl,
k) $R_3$, or
l) $-(CH_2)_n-NR_7R_8$;

$R_5$ is a hydrogen, $NO_2$, $NH_2$, $CF_3$, alkyl, aryl, -S-alkyl, -S-aryl or heteroaryl, -SO-alkyl or aryl, $-SO_2$-alkyl or aryl, or $R_3$;

$R_6$ is H, $CF_3$, alkyl, aryl or a pharmaceutically-acceptable counter-ion for carboxylic acids;

$R_7$ and $R_8$ are H, CO-alkyl, CO-aryl, alkyl, cycloalkyl, alkylaryl, aryl, heteroalkyl or aryl, or $R_7$ and $R_8$ can be taken together to form a piperidine ring or morpholine ring;

$R_9$ is alkyl or aryl;

$R_{10}$ is H, $CF_3$, alkyl, aryl or heteroaryl; and

n is 0-5 and p is 0-8; and

$\beta$ is

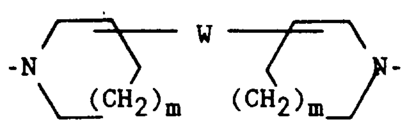

wherein W is $-(CH_2)_{1-6}-$ and m is 0-4;
and wherein "alkyl" has 1 to 8 C atoms, "aryl" has 6 to 12 C atoms and is optionally substituted by 1 to 3 substituents selected from OH, $C_{1-3}$ alkoxy, $C_{1-3}$ alkyl, $CF_3$, F, Cl and Br, "cycloalkyl" has 3 to 10 C atoms, and "heteroaryl" is aryl containing a heteroatom which is N, S or O.

**2.** A compound of claim 1, wherein $\beta$ is derived (by removing the 2 N-bound H atoms) from 1,3-bis(4-piperidinyl)propane or 1,2-bis(4-piperidinyl)ethane.

**3.** The compound of claim 1, which is 7,7'-[1,2-ethanediylbis(4,1-piperidinediylmethylene)]-bis[4,9-dimethoxy-5H-furo[3,2-g][1]benzopyran-5-one].

**4.** Use of a compound of any preceding claim, for the manufacture of a medicament for use in blocking or inhibiting ACAT enzyme.

**5.** Use of a compound of any preceding claim, for the manufacture of a medicament for use in preventing or treating atherosclerosis.

**Patentansprüche**

**1.** Verbindung, umfassend einen zweiwertigen Liganden $\alpha$-$\beta$-$\alpha$, wobei $\alpha$ strukturmäßig durch die Formel

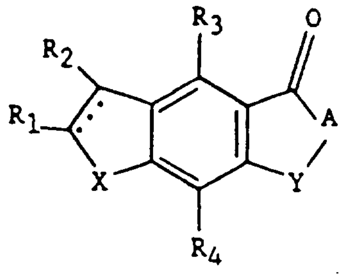

worin bedeuten:

X und Y unabhängig voneinander O oder S;

A C=CH-, N=C- oder $CR_5$=C-$(CH_2)_n$- verbunden mit *β*;

$R_1$ und $R_2$ unabhängig voneinander:

a) H,
b) Halogen,
c) Alkyl,
d) -$(CH_2)_p$-Aryl,
e) -$(CH_2)_p$-Heteroaryl,
f) -$(CH_2)_p$-$CO_2R_6$,
g) -$(CH_2)_p$-$CONR_7R_8$,
h) -$Si(R_9)$,
i) -$(CH_2)_n$-$NR_7R_8$,
j) -$(CH_2)_n$-$OR_{10}$,
k) -$CF_3$ oder
l) -$(CH_2)_n$-$SR_6$, -$(CH_2)_n$-$SOR_6$, -$(CH_2)_n$-$SO_2R_6$;

$R_3$

a) OH,
b) $OCH_2CH=CH_2$,
c) $OCH_2CH(OH)CH_2NHR_6$,
d) -O-Alkyl,
e) -O-$(CH_2)_n$-$CO_2R_6$ oder
f) -O-$(CH_2)_n$-$CONR_7R_8$;

$R_4$

a) Wasserstoff,
b) Halogen,
c) $NO_2$,
d) $NH_2$,
e) $CF_3$,
f) Alkyl,
g) Aryl,
h) -S-Alkyl oder -Aryl,
i) -SO-Alkyl oder -Aryl,
j) -$SO_2$-Alkyl oder -Aryl,
k) $R_3$ oder
l) -$(CH_2)_n$-$NR_7R_8$;

$R_5$ Wasserstoff, $NO_2$, $NH_2$, $CF_3$, Alkyl, Aryl, -S-Alkyl, -S-Aryl oder -Heteroaryl, -SO-Alkyl oder -Aryl, -$SO_2$-Alkyl oder Aryl oder $R_3$;

$R_6$ H, $CF_3$, Alkyl, Aryl oder ein pharmazeutisch akzeptables Gegenion zu Carbonsäuren;

$R_7$ und $R_8$ H, CO-Alkyl, CO-Aryl, Alkyl, Cycloalkyl, Alkylaryl, Aryl, Heteroalkyl oder -aryl oder $R_7$ und $R_8$ zusammen einen Piperidinring oder Morpholinring;

$R_9$ Alkyl oder Aryl;

$R_{10}$ H, $CF_3$, Alkyl, Aryl oder Heteroaryl; und

n = 0-5 und p = 0-8

wiedergegeben wird

und *β*

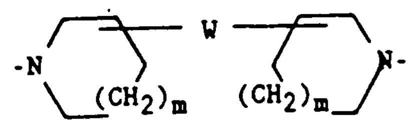

mit W gleich $-(CH_2)_{1-6}-$ und m = 0-4 entspricht und worin "Alkyl" 1 bis 8 C-Atom(e) enthält, "Aryl" 6 bis 12 C-Atome aufweist und gegebenenfalls durch 1 bis 3 Substituent(en), ausgewählt aus OH, $C_{1-3}$ Alkoxy, $C_{1-3}$ Alkyl, $CF_3$, F, Cl und Br, substituiert ist, "Cycloalkyl" 3 bis 10 C Atome besitzt und "Heteroaryl" für ein Heteroatom, nämlich N, S oder O, enthaltendes Aryl steht.

**2.** Verbindung nach Anspruch 1, worin $\beta$ von (durch Entfernen der 2 N-gebundenen H-Atome) 1,3-Bis-(4-piperidinyl)propan oder 1,2-Bis-(4-piperidinyl)-ethan abgeleitet ist.

**3.** Verbindung nach Anspruch 1, nämlich 7,7'-[1,2-Ethandiylbis-(4,1-piperidindiylmethylen)]-bis-[4,9-dimethoxy-5H-furo[3,2-g][1]benzopyran-5-on].

**4.** Verwendung einer Verbindung nach einem der vorhergehenden Ansprüche zur Herstellung eines Medikaments zur Verwendung bei der Blockade oder Hemmung von ACAT-Enzym.

**5.** Verwendung einer Verbindung nach einem der vorhergehenden Ansprüche zur Herstellung eines Medikaments zur Verwendung bei der Verhinderung oder Behandlung von Atherosklerose.

## Revendications

**1.** Composé comprenant : un ligand bivalent $\alpha$-$\beta$-$\alpha$ dans lequel $\alpha$ est représenté par la formule structurale

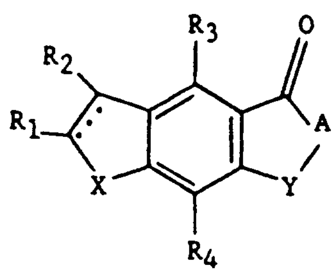

dans laquelle :

X et Y représentent, indépendamment O ou S ;

A représente un groupe C=CH-, N=C- ou $CR_5=C-(CH_2)_n$ et est lié à $\beta$ ;

$R_1$ et $R_2$ représentent, indépendamment :

a) H,
b) un groupe halogéno,
c) un groupe alkyle,
d) un groupe $-(CH_2)_p$-aryle,
e) un groupe $-(CH_2)_p$-hétéro-aryle,
f) un groupe $-(CH_2)_p-CO_2R_6$,
g) un groupe $-(CH_2)_p-CONR_7R_8$,
h) un groupe $-Si(R_9)$,
i) un groupe $-(CH_2)_n-NR_7R_8$,
j) un groupe $-(CH_2)_n-OR_{10}$,
k) un groupe $-CF_3$, ou
l) un groupe $-(CH_2)_n-SR_6$, $-(CH_2)_n-SOR_6$, $-(CH_2)_n-SO_2R_6$ ;

$R_3$ représente

a) un groupe OH,
b) un groupe $OCH_2CH=CH_2$,
c) un groupe $OCH_2CH(OH)CH_2NHR_6$,
d) un groupe -O-alkyle,
e) un groupe $-O-(CH_2)_n-CO_2R_6$, ou

f) un groupe $-O-(CH_2)_n-CONR_7R_8$ ;
$R_4$ représente
a) l'hydrogène,
b) un groupe halogéno,
c) un groupe $NO_2$,
d) un groupe $NH_2$,
e) un groupe $CF_3$,
f) un groupe alkyle,
g) un groupe aryle
h) un groupe -S-alkyle ou aryle,
i) un groupe -SO-alkyle ou aryle,
j) un groupe $-SO_2$-alkyle ou aryle
k) un groupe $R_3$, ou
l) un groupe $-(CH_2)_n-NR_7R_8$ ;

$R_5$ représente l'hydrogène, un groupe $NO_2$, $NH_2$, $CF_3$, alkyle, aryle, -S-alkyle, -S-aryle ou hétéroaryle, -SO-alkyle ou aryle, $-SO_2$-alkyle ou aryle, ou $R_3$ ;

$R_6$ représente H, un groupe $CF_3$, alkyle, aryle ou un ion complémentaire pharmaceutiquement acceptable pour des acides carboxyliques ;

$R_7$ et $R_8$ représentent H, des groupes CO-alkyle, CO-aryle, alkyle, cycloalkyle, alkylaryle, aryle, hétéroaryle ou aryle, ou bien $R_7$ et $R_8$ peuvent être réunis en formant un noyau pipéridine ou un noyau morpholine ;

$R_9$ représente un groupe alkyle ou aryle ;

$R_{10}$ représente H, un groupe $CF_3$, alkyle, aryle ou hétéro-aryle ; et

n a une valeur de 0 à 5 et p a une valeur de 0 à 8 ; et

$\beta$ répond à la formule

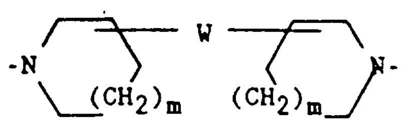

dans laquelle W représente un groupe $-(CH_2)_{1-6}$ et m a une valeur de 0 à 4 ;

le terme "alkyle" possédant 1 à 8 atomes de carbone, le terme "aryle" possédant 6 à 12 atomes de carbone et portant facultativement 1 à 3 substituants choisis entre des groupes OH, alkoxy en $C_1$ à $C_3$, alkyle en $C_1$ à $C_3$, $CF_3$, F, Cl et Br, le terme "cycloalkyle" possédant 3 à 10 atomes de carbone, et le terme "hétéro-aryle" désignant un groupe aryle contenant un hétéro-atome consistant en N, S ou O.

**2.** Composé suivant la revendication 1, dans lequel $\beta$ est dérivé (par élimination des 2 atomes de H liés à N) du 1,3-bis(4-pipéridinyl)propane ou du 1,2-bis(4-pipéridine)éthane.

**3.** Composé suivant la revendication 1, qui est la 7,7'-[1,2-éthanediylbis(4,1-pipéridinediylméthylène)]-bis-[4,9-diméthoxy-5H-furo[3,2-g][1]benzopyranne-5-one].

**4.** Utilisation d'un composé suivant l'une quelconque des revendications précédentes, pour la production d'un médicament destiné à être utilisé dans le blocage ou l'inhibition de l'enzyme ACAT.

**5.** Utilisation d'un composé suivant l'une quelconque des revendications précédentes, pour la production d'un médicament destiné à être utilisé dans la prévention ou le traitement de l'athérosclérose.